# EUROPÄISCHE PATENTANMELDUNG

(11) **EP 3 348 303 A2**
(43) Veröffentlichungstag der Anmeldung: **18.07.2018**
(21) Anmeldenummer: 17204715.1
(22) Anmeldetag: 30.11.2017
(51) Int. Cl.: A61M 35/00, A61L 26/00

(54) **FLÜSSIGPFLASTER, AUFTRAGEVORRICHTUNG UND DEREN VERWENDUNG**

(30) Priorität: 11.01.2017 DE 102017100443
(71) Anmelder: Elixmann, Madeleine, 74078 Heilbronn (DE)
(72) Erfinder: Elixmann, Madeleine, 74078 Heilbronn (DE)
(74) Vertreter: Pöhner, Wilfried Anton

(57) **Zusammenfassung**

Auftragevorrichtung (1) und Flüssigpflaster (2) umfassend eine oder mehreren Komponenten, wobei das Flüssigpflaster nach Auftragen auf eine Hautstelle eines Anwenders durch passive oder aktive Aushärtung einen geschlossenen Film bildet und in seinen rheologischen Eigenschaften auf die Auftragevorrichtung abgestimmt ist, die Auftragevorrichtung umfassend ein Reservoir (3) mit Flüssigpflaster (2) aus festem, durch keine der Inhaltsstoffe des Flüssigpflasters angreifbarem Material, insbesondere Glas oder Kunststoff, wobei das Reservoir als ein Hohlkörper mit einer Öffnung (31) ausgebildet ist, wobei auf der Öffnung (31) des Reservoirs (3) dem ein Auftragemittel (4) mit Auftragespitze (42) befestigt ist, welches dazu geeignet ist, das Flüssigpflaster (2) gleichmäßig auf die Hautstelle des Anwenders aufzutragen und zu verteilen.

## Beschreibung

Vorliegende Erfindung befasst sich mit einer Auftragevorrichtung für Flüssigpflaster umfassend ein Flüssigpflaster aus einer oder mehreren Komponenten, wobei das Flüssigpflaster nach Auftragen auf eine Hautstelle eines Anwenders durch passive oder aktive Aushärtung einen geschlossenen Film bildet und in seinen rheologischen Eigenschaften auf die Auftragevorrichtung abgestimmt ist, ein Reservoir für das Flüssigpflaster aus festem, durch keine der Inhaltsstoffe des Flüssigpflasters angreifbarem Material, wobei das Reservoir als ein Hohlkörper mit einer Öffnung ausgebildet ist, sowie einem auf die Auftragevorrichtung abgestimmten Flüssigpflaster und deren gemeinsame Verwendungen.

Flüssigpflaster zum Verschließen von Wunden sind seit einiger Zeit bekannt. Es handelt sich dabei üblicherweise um eine Mischung mehrerer Substanzen, welche zumindest einem filmbildenden Polymer umfasst, welches in einem Lösungsmittel gelöst ist. Nach dem Auftragen auf eine verletzte Hautstelle verdunstet das Lösungsmittel und lässt einen mehr oder minder stark vernetzten Polymerfilm zurück, welcher die Wunde verschließt.

Die Vorteile von Flüssigpflastern gegenüber konventionellen textilen Pflastern liegen auf der Hand. Zum einen lassen sie sich problemlos auf Größe und, je nach Auftrageart, auch die Form der Wunde anpassen, wohingegen man bei konventionellen Pflastern eine Auswahl verschiedener Größen vorrätig halten muss.
Weiterhin sind Flüssigpflaster schnell zu applizieren. Sie werden im Stand der Technik entweder in einer Sprühflasche angeboten oder, wenn sie sich aufgrund hoher Viskosität nicht zum Versprühen eignen, in einem per Deckel verschlossenen Gefäß verkauft. In letzterem Fall kann man das Flüssigpflaster mit einem Pinsel, insbesondere einem nach Art eines Nagellackfläschchens in den Deckel des Gefäßes integrierten Pinsel, entnehmen und auf die gewünschte Stelle auftragen kann. Im Falle eines Sprühpflasters dauert das Auftragen selbst nur wenige Sekunden, die Trocknung aufgrund der häufig verwendeten schnellflüchtigen Lösungsmittel in der Größenordnung weniger zehn Sekunden bis eine Minute. Etwas länger dauert das Auftragen und Aushärten dickflüssigerer Flüssigpflaster mittels Pinsel. Darüber hinaus bietet sich hier der Vorteil, dass der wundverschließende Pflasterfilm nicht nur an die Größe, sondern auch an die Form der Wunde angepasst werden kann.

Als weiterer Vorteil ist bei dickflüssigeren, per Pinsel aufzutragenden Pflastern zu verzeichnen, dass durch Bereitstellen von, möglicherweise mehreren unterschiedlichen, farbigen Pflastern eine individuelle Gestaltung des wundverschließenden Pflasterfilms möglich ist. Bei Sprühpflastern ist dies aufgrund der niedrigen Viskosität und der üblichen, großflächig sprühenden Düsen nicht durchführbar.

Als einzige Nachteile von Flüssigpflastern gegenüber konventionellen, textilen Pflastern ist das erhöhte Gewicht pro Wundfläche sowie die potentielle Hautunverträglichkeit der verwendeten Substanzen, insbesondere der Lösungsmittel, zu verzeichnen.

Die Anmeldeschrift US 2015/0343112 A1 offenbart beispielsweise ein farbiges Flüssigpflaster zur Behandlung von Verletzungen wie Niednägeln oder Fleischwunden, welches als Filmbildner Schellack und/oder Zelluloseacetat, gelöst durch eine Kombination der Lösungsmittel Dichlormethan (Dielektrizitätskonstante 9,1, Dipolmoment 1,60 Debye) und Ethanol (Dielektrizitätskonstante 25, Dipolmoment 1,67 Debye). Beide können in erhöhter Konzentration Hautreizungen hervorrufen, insbesondere bei empfindlichen Personen.
Ein spezielles Mittel zum Auftragen ist der Anmeldeschrift nicht zu entnehmen, jedoch wird das vom Anmelder vertriebene Produkt in nagellackfläschchenartigen Behältnis mit einem in den Deckel integrierten Pinsel angeboten. Bei der Anwendung bestehen darum die gleichen Nachteile wie bei der Anwendung von Nagellack: bei jeder Verwendung muss der Deckel abgeschraubt und das Behältnis also für eine gewisse Zeit geöffnet werden. Dabei kann Stück für Stück das im Flüssigpflaster enthaltene Lösungsmittel entweichen, so dass das Flüssigpflaster mit der Zeit immer dickflüssiger und schlechter verteilbar wird. Im Extremfall kann es dadurch soweit eintrocknen, dass es nicht mehr verwendbar ist. Darüber hinaus muss der Anwender, falls keine Abstellmöglichkeit vorhanden ist, das offene Fläschchen in der Hand balancieren während er sich auf das gleichmäßige Verteilen des Flüssigpflasters konzentrieren will. Ferner wird der Pinsel hierbei vollständig in das Flüssigpflaster eingetaucht. Nach dem Herausziehen muss daher zunächst überschüssiges Pflaster abgestreift werden. Insoweit dies nicht erfolgreich ist, können beim Auftragen unerwünschte Pflasterflecken entstehen oder die gewünschte Größe und Form des Flüssigpflasters lassen sich nicht genau erreichen.

Vor diesem Hintergrund hat sich vorliegende Erfindung die Aufgabe gestellt, eine Vorrichtung zum Auftragen von Flüssigpflastern sowie ein dazu passend abgestimmtes Flüssigpflaster zu finden, die ein schnelles und präzises Auftragen ermöglichen, dabei lange verwendbar sind ohne einzutrocknen und eine sehr gute Hautverträglichkeit aufweisen.

Gelöst wird diese Aufgabe durch die Auftragevorrichtung gemäß Anspruch 1 in welcher das Flüssigpflaster nach Anspruch 7 gemäß der Verfahren der Ansprüche 10 oder 11 verwendet wird.

Die wesentlichen Merkmale der erfindungsgemäßen Auftragevorrichtung sind das Reservoir, welches mit erfindungsgemäßem Flüssigpflaster befüllt ist, und ein auf einer Öffnung des Reservoirs dicht schließend aufgesetztes Auftragemittel, welches zum gezielten Auftragen des Flüssigpflasters eine Auftragespitze umfasst. Das Auftragemittel ist innen hohl verfügt über einen oder mehrere Kanäle, durch welche das Flüssigpflaster bei Schräghalten oder auf dem Kopf stellen der Auftragevorrichtung zur Auftrageöffnung des Auftragemittels und von dort in die Auftragespitze fließen kann.

Das Reservoir ist ein Hohlgefäß mit mindestens einer Öffnung, bevorzugt an einem oberen Ende, auf welche das Auftragemittel flüssigkeits- und gasdicht befestigt ist, beispielsweise per Schraub-, Dreh-, Steck- oder Bajonettverschluss. Um eine weitergehende Dichtigkeit gegen Verdunsten von Substanzen aus dem Flüssigpflaster, insbesondere eventuell verwendete Lösungsmittel, zu erreichen kann zwischen dem Rand der Öffnung des Reservoirs und dem Auftragemittel eine Dichtung, beispielsweise in Form eines Dichtringes, vorgesehen sein. Die äußere Form sowie Gewicht sind für ein leichtes einhändiges Halten konzipiert und ergonomisch optimiert. Im einfachsten Fall ist es ein Hohlzylinder welcher an einem unteren Ende mittels einer Bodenfläche verschlossen ist.

Im Inneren des Reservoirs kann das Flüssigpflaster entweder in einer einzelnen Kammer aufbewahrt sein, oder es sind mehrere Kammern vorhanden. Dies ist z.B. zusammen mit der Verwendung von mehrkomponentigen Flüssigpflastern, bei denen die Filmbildende Wirkung erst nach Mischung der Komponenten eintreten kann nötig, damit die Komponenten getrennt voneinander untergebracht werden können. Dann sind entsprechend mehrere Öffnungen am oberen Ende des Reservoirs sowie eine gleiche Anzahl diesen Öffnungen zugeordneter Zuleitungskanäle zur Auftragespitze erforderlich. Die Mischung der Komponenten erfolgt erst kurz vor dem Auftragen in der Auftragespitze. Dies ist effektiver als eine Mischung erst auf der Haut, bei welcher die korrekte, gleichmäßige Durchmischung der Komponenten fraglich wäre, stellt aber auch erhöhte Anforderungen an die Reinigbarkeit der Auftragespitze von eingetrocknetem Flüssigpflaster.

Mehrere Kammern können aber auch bei Verwendung Flüssigpflastern sinnvoll sein, bei der jede Komponente für sich in den funktionellen Inhaltsstoffen übereinstimmen, insbesondere also auch als einkomponentiges Flüssigpflaster funktionieren und aushärten können, wenn sich die einzelnen Komponenten in sekundären Eigenschaften wie Farbe oder Geruch unterscheiden. In Verbindung mit einer Auftragespitze, welche selektiv mit einer der Reservoirkammern in fluide Kommunikation gebracht werden kann, z.B. durch Verdrehen des Deckels um einen entsprechenden Winkel, kann der Anwender so mittels der erfindungsgemäßen Auftragevorrichtung ästhetisch ansprechende, mehrfarbige, strukturierte Flüssigpflasterfilme oder solche mit verschiedenen Duftnoten kreieren.

Das erfindungsgemäße Flüssigpflaster zur Verwendung mit der erfindungsgemäßen Auftragevorrichtung hat mindestens eine Komponente, kann aber auch aus mehreren bestehen. Jede Komponente ist hierbei eine flüssige Mischung von Flüssigkeiten und/oder Feststoffen, kann aber auch ausschließlich aus einer Substanz bestehen. Als Filmbildner kommen verschieden Polymere in Frage. Die gewünschten Eigenschaften des ausgehärteten Films sind Festigkeit, Flexibilität, Wasserdichtigkeit bei gleichzeitiger Gasdurchlässigkeit und weiterhin eine feste, dauerhafte Anhaftung besonders an den Rändern, um eine Ablösung von dort aus vorzubeugen.
Dabei kann die filmbildende Vernetzung durch chemische Reaktionen, d.h. Ausbildung kovalenter Bindungen zwischen den Filmbildenden Molekülen zustande kommen. Wegen der hohen Aktivierungsenergie ist dies jedoch weitgehend nur in Verbindung mit einer aktiven Aushärtung z.B. durch Lichteinstrahlung, oder bei Aktivierung durch weitere Substanzen, sogenannten Härtern umsetzbar. Obwohl dies beispielsweise für stationäre Anwendungen in Krankenhäusern oder Arztpraxen durchaus interessant sein kann, ist für die überwiegenden Fälle in der Praxis ein Flüssigpflaster mit passiver Aushärtung zu bevorzugen.

Diese Eigenschaften lassen sich durch verschiedene Polymere realisieren. Bei der konkreten Auswahl ist zu beachten, dass Festigkeit und Härte des Films weitgehend durch den Vernetzungsgrad bestimmt werden. Je höher dieser ist, desto fester und härter, aber auch weniger flexibel und elastisch ist ein Polymerfilm. Vernetzung bedeutet hierbei die Zahl der Bindungen mit anderen Molekülen pro Molekül. Diese Bindungen können ionische, Wasserstoffbrücken oder kovalente Bindungen sein.
Erstere könnten beispielsweise durch Funktionalisierung, d.h. Substituierung von Wasserstoffatomen durch größere Seitenmoleküle, der Polymerketten mit organischen Salzen wie Carbonaten erreicht werden, spielen jedoch eher eine untergeordnete Rolle. Wasserstoffbrückenbindungen entstehen zwischen den stark elektropositiven Wasserstoffatomen von Hydroxylgruppen der Polymere als Donatoren und elektronegativen Sauerstoffatomen als Akzeptoren. Sie stellen den häufigsten vernetzenden Bindungstyp bei elastischen Filmen dar. Kovalente Bindungen, bei denen sich zwei oder mehr Atome Elektronen in gemeinsamen (Molekül-) Orbitalen teilen, sind die stabilsten, benötigen zur Herstellung jedoch in der Regel auch wesentlich mehr Aktivierungsenergie als Wasserstoffbrücken, welche spontan eingegangen werden können.
Als zusätzliche zusammenhaltende Kräfte sind ferner die Van-der-Waals Wechselwirkungen allgegenwärtig. Da sie nicht punktuell zwischen zwei (oder einer kleinen Zahl) von Atomen, sondern zwischen den Molekülen als Ganzes Wirken, werden sie üblicherweise nicht als Bindungen als solche verstanden. Auch spielen sie zwar in temporären, leicht vernetzten Polymerfilmen noch eine Rolle, d.h. z.B. auch während und vor der Aushärtung der hier gewünschten Filme. Beim fertig ausgehärteten Film mit der hier gewünschten Festigkeit wird ihr Beitrag jedoch von den zuvor beschriebenen Bindungen dominiert.

Um den für einen wundschützenden Film optimalen Kompromiss zwischen Festigkeit und Elastizität zu finden, müssen also die (mittlere) Kettenlänge, der Vernetzungsgrad, d.h. Zahl der Bindungen, und die Relation der starken kovalenten zu den schwächeren Wasserstoffbrückenbindungen variiert werden. Dabei ist auch zu bedenken, dass Wasserstoffbrücken und die dafür benötigten funktionalen Gruppen tendenziell die Angreifbarkeit des Films durch Wasser erhöhen.

Ersteres kann zum einen durch die Molekülgröße und zum anderen die Verästelung geändert werden. Eine höhere Verästelung lässt sich erreichen, indem an Polymere mit zunächst wenigen oder keinen Seitenketten, mehr oder weniger lange Seitenketten anreagiert werden. Dies können im einfachsten Fall Alkylgruppen sein. Da diese kein Sauerstoffatom enthalten würden dann aber nur die Van-der-Waals-Kräfte gesteigert, Wasserstoffbrückenbindungen könnten keine entstehen. Es wäre nur denkbar, eine Vernetzung durch kovalente C-C Bindungen herzustellen, was aber sehr hohe Aktivierungsenergie erfordern wurde und bei einer Anwendung auf der Haut schwierig ist. Einfache wäre dies bei Verwendung von Polymeren mit C=C Doppelbindungen oder Dreifachbindung (Alkene oder Alkine) oder der Funktionalisierung von (langkettigen) Alkanen mit Alkenyl oder Alkinyl Seitengruppen, da Doppel- und insbesondere Dreifachbindungen mit weniger Aktivierungsenergie aufgebrochen werden können.
Um die Möglichkeit der Vernetzung durch Wasserstoffbrückenbindungen zu schaffen können Polymere, die Hydroxyl oder Carboxyl-Reste enthalten verwendet werden, oder entsprechende funktionale Gruppen an Hydroxyl/Carboxyl freie bzw. arme Polymere angeschlossen werden. Die Funktionalisierungsreaktionen gehören hierbei zum Herstellungsprozess des Flüssigpflasters, und passieren nicht erst bei dessen Anwendung. Es sind hier zwei unabhängige, oder zumindest nicht äquivalente, Parameter zu beachten: die (mittlere) Zahl der Sauerstoffatome pro Polymerkette sowie Dichte der Sauerstoffatome. Vereinfacht gesprochen führt eine hohe Dichte zu einer hohen Zahl an Wasserstoffbrücken, der Vernetzungsgrad kann dabei jedoch dennoch relativ niedrig bleiben, falls vergleichsweise kurzkettige Polymere verwendet sind. Für die Festigkeit des ausgehärteten Films bedeutet dies, dass diese tendenziell niedriger ist, als bei gleicher Sauerstoffdichte, aber längerkettigen Polymeren.

Der weitere erwähnte Parameter, das Verhältnis der Wasserstoffbrücken zu den Kovalenten Bindungen, ist als Zahl der Bindungen pro Volumen zu verstehen und kann daher durch die Wahl der mittleren Kettenlänge sowie die Dichte der Sauerstoffatome (Dichte der Carboxyl oder Hydroxyl-Gruppen) beeinflusst werden.

Teilorganische oder anorganische funktionale Gruppen oder Polymere, insbesondere auf Silizium-Basis, wie Carbosilane oder Siloxane, sind in diesem Zusammenhang ebenfalls erwägenswert. Vorliegende Erfindung schlägt insbesondere funktionalisiertes Polydimethylsiloxan als Filmbildner in mindestens einer Komponente des erfindungsgemäßen Flüssigpflasters vor. Als funktionale Gruppen, welche einen Teil der Methylgruppen substituieren, kommen langkettige Alkohole oder Alkenole zur Erhöhung des Verästelungsgrades der Polymere in Frage, mit eventueller zusätzlicher Hydroxylierung zur Erhöhung der Dichte der Wasserstoffbrückenbindungen, d.h. Zahl der Wasserstoffbrücken pro Volumen. Polysiloxan-basierte Polymere bieten die Vorteile hoher Hautverträglichkeit und einer einfachen Synthetisierung. Die Benetzungseigenschaften hängen von Art und Anzahl der funktionalen Gruppen, aber noch mehr vom verwendeten Lösungsmittel ab. Sind polare Gruppen, wie Hydroxyl, Carboxyl oder Siloxyl, in nicht zu großer Zahl vorhanden, entsteht nach Verdunstung des verwendeten Lösungsmittels oder nach aktiver Aushärtung, z.B. durch Lichteinstrahlung oder Hinzufügen eines Härters, ein wasserfester, aber wasserdampf- und sauerstoffdurchlässiger Film, der sich durch hervorragende Elastizität und ein samtiges Hautgefühl hervorhebt.

Falls eine rein passive Aushärtung gewünscht ist, wäre die einfachste Umsetzung derart, dass der Filmbildner in einem Lösungsmittel gelöst werden. Hierfür bieten sich in Verbindung mit einem Polysiloxan als Filmbildner zyklische Siloxane wie Cyclotetrasiloxan, Cyclopentasiloxan oder Cyclohexasiloxan an, da sie den Siloxanpolymeren ähnliche chemische Eigenschaften haben, und sich nur in den gewünschten physikalischen Eigenschaften unterscheiden, d.h. bei Raumtemperatur und Normaldruck flüssig sind. Andere Lösungsmittel mittlerer Polarität wie Methanol, Ethanol, Propanol oder Dimethylester, oder, als eine aprotische, kaum polare Ergänzung, Ethylacetat, kommen ebenfalls in Frage. Im Sinne einer erwünschten zügigen Aushärtung des erfindungsgemäßen Flüssigpflasters ist ein leichtflüchtiges Lösungsmittel zu bevorzugen. Insbesondere wird ein Lösungsmittel bevorzugt, dessen Flüchtigkeit, d.h. Verdunstungsrate bzw. Dampfdruck, sich im Temperaturbereich von üblicher Raumtemperatur (ca. 20Grad Celsius) und Hauttemperatur (ca. 35-37Grad Celsius) möglichst stark steigert. Dadurch ist erreicht, dass das Eintrocknen einer bei Raumtemperatur gelagerten erfindungsgemäßen Auftragevorrichtung zusätzlich zur Verwendung einer dicht schlie-ßenden Kappe auch durch die Eigenschaften des Lösungsmittels hinausgezögert wird. Darum werden insbesondere Methanol, Ethanol oder Propanol zusammen mit Ethylacetat als Lösungsmittel empfohlen.

Mehrere Vorteile der erfindungsgemäßen Kombination von Auftragevorrichtung und Flüssigpflaster sind leicht nachvollziehbar.

Der Größte und Einleuchtendste ist wohl die schnelle und einfache Verwendung. Ist ein ausreichend befülltes erfindungsgemäße Auftragevorrichtung zur Hand, ist eine Wunde schnell versorgt. Nachdem Reinigung und Desinfektion erfolgt sind, benötigt das Aufbringen eines Flüssigpflasterfilms nur wenige Sekunden. Dazu wird die Auftragevorrichtung in ein Hand genommen, ggf. gut geschüttelt, um alle Komponenten des Flüssigpflasters wieder zu Homogenisieren, dann wird die Kappe abgenommen, die Auftragevorrichtung mit der Auftragespitze nach unten gehalten und Letztere über die zu versorgende Stelle gebracht. Schwerkraftgetrieben oder durch leichte Druckausübung auf das Reservoir fließt Flüssigpflaster aus der oder den Reservoirkammern durch das Auftragemittel in die Auftragespitze, wo sich ggf. die verschiedenen Komponenten begegnen und durchmischen. Durch leichtes Aufdrücken der Spitze und/oder Druck mit der Hand auf das Reservoir fließt das, ggf. gemischte, Flüssigpflaster aus der Spitze auf die zu Haut des Anwenders. Bei einer sehr fließfähigen Flüssigpflastermischung bildet sich nun von selbst ein die Hautstelle bedeckender Film. Bevorzugt ist jedoch ein Flüssigpflaster mit mäßiger bis geringer Fließfähigkeit, so dass der Anwender selbst durch entsprechende Bewegung der Auftragespitze das Pflaster verteilt und so dessen Form und Größe sehr genau einstellen kann. Eine gewisse Fließfähigkeit ist jedoch in jedem Fall nötig, damit sich der Film nach der manuellen Verteilung noch vor dem Aushärten zu einer gleichmäßigen Dicke nivelliert.
Da das erfindungsgemäße Flüssigpflaster eine vergleichsweise hohe Viskosität aufweist, dauert es bis zur Aushärtung zu einem flexiblen, anschmiegsamen Film, abhängig von Haut- und Umgebungstemperatur und Luftbewegung, nur wenige zehn Sekunden bis etwa einer Minute.

Hierbei kommt Vorteilhaft zum Tragen, dass das Flüssigpflaster bis zum Zeitpunkt, zu dem es konkret auf die Wunde aufgebracht wird, in der Auftragevorrichtung luftdicht eingeschlossen bleibt. Zum einen kann dadurch weniger Lösungsmittel verwendet werden, da nicht die Gefahr des baldigen Eintrocknens bei häufiger Verwendung besteht. Weniger Lösungsmittel führt zu einem dickflüssigeren Flüssigpflaster höherer Viskosität mit den oben angedeuteten Vorteilen einer akkurateren präziseren Auftragung und schnelleren Aushärtung. Die Auftragung des erfindungsgemäßen Flüssigpflasters mittels der erfindungsgemäßen Auftragevorrichtung erfolgt in einer an das Schreiben/Malen mit einem Stift oder Marker erinnernden Art und Weise, die demgemäß für die große Mehrzahl der Menschen sehr einfach und intuitiv ist. Hierdurch ist es auch ermöglicht, dass bei Verwendung von Flüssigpflastern mit verschiedenen Farben, entweder mittels mehrerer, separater erfindungsgemäßer Auftragevorrichtungen oder in einer mehrkammerigen Auftragevorrichtung, bei der die aktive Kammer selektierbar ist, der Anwender einfach und schnell einen optisch ansprechenden Pflasterfilm mit gewünschten Motiven oder Strukturen schaffen kann.

Die Verwendung bei erneuter Überdeckung einer bereits behandelten Stelle, also der Wechsel des Flüssigpflasters

Vorteilhafte Weiterbildungen, welche einzeln oder in Kombination realisierbar sind, sofern sie sich nicht gegenseitig offensichtlich ausschließen, sollen im Folgenden näher beschrieben werden.

Bevorzugt ist das Auftragemittel der erfindungsgemäßen Auftragevorrichtung als Hohlkörper mit einem oberen und einem unteren Ende ausgebildet, wobei es mit seinem unteren Ende auf der Öffnung des Reservoirs aufgesetzt und befestigt ist. Am oberen Ende befindet sich die Auftrageöffnung und jenseits dieser sitzt die Auftragespitze. Der Querschnitt des Auftragemittels ist besonders bevorzugt in etwa kreisförmig und entweder konstant oder sich nach oben hin verringernd, so dass zumindest der sich von der oberen Auftrageöffnung bis zum Beginn der Befestigungseinrichtung am unteren Ende erstreckende Korpus die Form eines Hohlzylinders oder eines hohlen Kegelstumpfes hat. Das Auftragemittel kann unlösbar mit der Öffnung des Reservoirs verbunden sein, etwa durch Verkleben oder Verschweißen. Bevorzugt ist es jedoch lösbar verbunden, beispielsweise mittels Schraub-, Dreh-, Steck- oder Bajonettverschluss.

Die Auftragespitze ist bevorzugt am oberen Ende von etwa semisphärischer, ovaler oder spitz zulaufender Form ist und umfasst bevorzugt einen Schwamm bzw. eine schwammartig poröse oder faserige und elastische Struktur. Sie kann auch aus einem Pinsel mit mittelfeinen Borsten bestehen. In einer anderen Ausführungsform umfasst die erfindungsgemäße Auftragespitze eine Kugel, welche in einer komplementären Aussparung in der Auftrageöffnung des Auftragemittels festgehalten wird und dabei drehbar gelagert ist.
Das Material der Auftragespitze weist bevorzugt eine geringe Anhaftung des ausgehärteten Flüssigpflastermaterials auf, so dass die Reinigung erleichtert wird. Dies kann dadurch realisiert sein, dass die Auftragespitze insgesamt aus einem wenig haftenden Material besteht, oder aber speziell mit nichthaftendem Material beschichtet ist, wie beispielsweise Polytetrafluoräthylen oder Polyhalogenolefine ähnlicher Eigenschaften.

Das Reservoir der erfindungsgemäßen Auftragevorrichtung hat bevorzugt entweder eine einzige Kammer oder verfügt in seinem inneren über mehrerer Kammern. Letzteres ermöglicht die Verwendung mit mehrkomponentigen Flüssigpflastern, die erst nach Mischung ihre pflasterfilmbildenden Eigenschaften erhalten, oder auch die Verwendung mit mehreren einkomponentigen Flüssigpflastern verschiedener Farbe. Ein mehrkammeriges Reservoir weist bevorzugt für jede Kammer eine eigene Öffnung auf, so dass eine verfrühte Durchmischung von Komponenten vermieden wird.

Vorliegende Erfindung schlägt weiterhin bevorzugt vor, das Reservoir aus einem zumindest teilweise Transparenten Material zu fertigen oder mit transparenten Ausschnitten zu versehen. Dies erlaubt es, Farbe und Füllstand des Flüssigpflasters direkt zu erkennen. Dies wird durch Verwendung von Glas oder einem transparenten Kunststoff wie Acrylglas, Polycarbonat oder Polyethylen erreicht Das Material sollte in besonders bevorzugten Ausführungsformen weiterhin aber auch flexibel und elastisch sein, so dass durch Druckausübung auf das Reservoir die Ausflussrate dosiert werden kann und außerdem Bruchfestigkeit garantiert ist. Dies ist bei Reservoiren aus Kunststoff der Fall. Der Kunststoff muss jedoch gegen die im Flüssigpflaster verwendeten Substanzen, insbesondere die Lösungsmittel, resistent sein. Bevorzugt sind aus diesen Gründen Polycarbonat oder Polyethylen.

Das erfindungsgemäße, auf die Eigenschaften der Auftragevorrichtung abgestimmte Flüssigpflaster besteht entweder aus genau einer Komponente oder aus mehreren Komponenten. Im ersten Fall liegen alle das Flüssigpflaster bildenden Substanzen in Lösung und/oder Suspension vor. Dies umfasst in jedem Fall mindestens einen Filmbildner. Bevorzugt ist dies ein funktionalisiertes Polyethylen oder Siloxan, insbesondere funktionalisiertes Dimethylsiloxan. Als funktionale Gruppen kommen besonders bevorzugte einfache oder mehrfache Alkanole, Alkenole oder Alkinole sowie Hydroxyl, Carboxyl oder Siloxyl zum Einsatz.

Das oder die verwendeten Lösungsmittel sind bevorzugt leicht flüchtig und weisen einen große Differenz des Dampfdruckes bei Raumtemperatur im Vergleich zur Hauttemperatur auf, beispielsweise dadurch, dass der Siedepunkt nicht weit über der Hauttemperatur liegt. Weiterhin sollen die Lösungsmittel eine geringe Toxizität und gute Hautverträglichkeit aufweisen. Der Polarität der darin zu lösenden Polymere entsprechend ist die Polarität des Lösungsmittels bevorzugt im mittleren Bereich, d.h. weder vollkommen unpolar noch von einer mit Wasser vergleichbaren Polarität. Vorliegende Erfindung schlägt daher Ethylacetat, Methanol, Ethanol, Propanol, Cyclotetrasiloxan, Cyclopentasiloxan oder Cyclohexasiloxan als Lösungsmittel vor. Da die Vernetzung des Ausgehärteten Films zum Großteil auf Wasserstoffbrückenbindungen basiert, und dementsprechend der Filmbildner eine vergleichsweise hohe dichte an Sauerstoffatomen als Akzeptoren und Wasserstoffatomen in Hydroxylgruppen als Donatoren besitzt, ist der Einsatz eines aprotischen Lösungsmittels zumindest als Hauptlösungsmittel vorzuziehen. Ethylacetat ist ein solches aprotisches Lösungsmittel, welches darüber hinaus, wie erwähnt, eine niedrige Toxizität und gute Hautverträglichkeit aufweist. Da Ethylester jedoch auch eine recht geringe Polarität besitz (Dipolmoment 1.78 Debye, Dielektrizitätszahl 6,0) ist eventuell zur besseren Löslichkeit des vergleichsweise polaren Polymers zusätzlich ein weiteres, stärker polares Lösungsmittel einzusetzen. Dies verbessert außer der Löslichkeit auch die Benetzung feuchter Hautstellen, was bei der Wundbehandlung sehr wichtig ist.

Vorliegende Erfindung schlägt insbesondere vor, Ethylacetat und Ethanol oder Propanol in Verhältnissen zwischen 3:1 bis 1:2 vermischt einzusetzen.

Weiterhin schlägt vorliegende Erfindung in einer weiteren Ausgestaltung vor, in eine Komponente des Flüssigpflasters Mikro- oder Nanopartikel einzubringen, welche zum einen die rheologischen Eigenschaften Fließfähigkeit und Benetzung weiter verbessern, insbesondere die Oberflächenspannung herabsetzen, und zum anderen für eine ausreichende luft- bzw. Gasdurchlässigkeit des ausgehärteten Films sorgen. Dazu wird vorgeschlagen, Partikel zu verwenden, die sich bei steigender Konzentration in gewissem Rahmen zu größeren, mikroporösen Komplexen zusammenlagern, um die Struktur des sich bildenden Filmes aufzulockern. Die Partikel selbst können zusätzlich ebenfalls eine poröse bzw. gasdurchlässige Struktur aufweisen. Es ist ferner von Vorteil, wenn die Partikel anti-bakterielle Eigenschaften haben. Als Mikro- oder Nanopartikel mit diesen gewünschten Eigenschaften kommen beispielsweise pyrogene Kieselsäure, Zinkoxid-, Aluminiumoxid- oder Silber-Nanopartikel.

Die Heterogenität und Luftdurchlässigkeit des ausgehärteten Films kann auch durch die Verwendung zweier Lösungsmittel verbessert werden, wenn diese deutlich unterschiedliche Polaritäten und/oder Siedepunkte aufweisen. Bei genügend unterschiedlicher Polarität ist eine vollständige Mischbarkeit der Lösungsmittel nicht mehr gegeben. Vorliegende Erfindung schlägt vor eine Suspension aus zwei solchermaßen nicht mischbarer Lösungsmittel zu verwenden, wobei der Filmbildner in dem ihm chemisch ähnlicheren, d.h. ihn besser lösenden Lösungsmittel gelöst wird, bevor eine kleinere Menge des zweiten Lösungsmittels möglichst fein einsuspergiert wird. Bei der Aushärtung vernetzt sich der Filmbildner bevorzugt an den Grenzflächen zwischen den Lösungsmitteltröpfchen und bildet so deutlich heterogenere Strukturen als bei Aushärtung mit nur einem Lösungsmittel. Dieser Effekt wird noch verstärkt, wenn die beiden Lösungsmittel deutlich unterschiedliche Siedepunkte bzw. effektive Dampfdrücke bei Hauttemperatur aufweisen. Effektiver Dampfdruck bezeichnet hierbei die Differenz aus absolutem Dampfdruck des Lösungsmittels im (noch flüssigen) Film und dem Dampfdruck des Lösungsmittels in der Umgebungsluft, wobei letzterer hauptsächlich bei Verwendung von Wasser als einer Lösungsmittelkomponente eine Rolle spielt. Sind die Siedepunkte sehr unterschiedlich, so kann auch bei vollständiger Mischbarkeit der beiden Lösungsmittel ein auflockernder Effekt dadurch erreicht werden, dass die eine, bevorzugt die höher konzentrierte, Lösungsmittelkomponente wesentlich schneller verdunstet als die andere, wobei letztere dann temporär Einlagerungen innerhalb des sich vernetzenden Polymerfilms bildet, bevor es auch verdunstet. Dies setzt voraus, dass die Affinität des Filmbildners zu dem einen Lösungsmittel wesentlich größer ist als zu dem anderen.
Als mögliche Lösungsmittelkombination schlägt vorliegende Erfindung eine Suspension aus Ethylacetat und Wasser im Verhältnis 2:1 bis 20:1, bevorzugt 5:1 bis 10:1, alternativ eine Mischung aus Ethylacetat und 1-Propanol oder Diethylether und Ethanol jeweils im Verhältnis 5:1 bis 10:1 vor.

Das erfindungsgemäße Flüssigpflaster hat bevorzugt eine eher hohe Viskosität und fließt damit nur langsam, was das gezielte und präzise Auftragen vereinfacht. Nichtsdestotrotz soll aber eine gute Benetzung trockner, talgiger und insbesondere feuchter Hautstellen gegeben sein. Durch Wahl der Lösungsmittel und eine entsprechende Lösungsmittelkonzentration werden die Viskosität und Benetzungseigenschaften des erfindungsgemäßen Flüssigpflasters im relevanten Temperaturbereich zwischen 20-40 Grad Celsius wie gewünscht eingestellt. Bevorzugt ist hierbei eine Viskosität zwischen 0,2 bis 20, besonders bevorzugt zwischen 1 bis 5 Pascalsekunden. Für eine gute Benetzung sollte die Oberflächenspannung weniger als 40, besonders bevorzugt zwischen 10 und 30 Millinewton pro Meter betragen.

Zusätzlich zu den wesentlichen funktionalen Substanzen des erfindungsgemäßen Flüssigpflasters können auch weitere Stoffe, etwas solche mit antiseptischen oder wundheilungsfördernden Eigenschaften vorhanden sein. Die Lösungsmittel selbst wirken oft bereits antiseptisch, etwa im Falle von Methanol, Ethanol oder Propanol, verdunsten aber sehr schnell und können dann somit nicht mehr weiter wirken. Darum schlägt vorliegende Erfindung für eine dauerhafte antiseptische bzw. antibakterielle Wirkung vor, Chlorhexidin in Konzentrationen zw. 0.1 - 1 % mit zuzugeben. Zur Wundheilungsförderung wird bevorzugt Dexpanthenol und/oder Aloe Vera und/oder Ringelblumenextrakt und/oder Kamillenblütenextrakt und/oder Extrakt des roten Sonnenhutes mit zugegeben, wobei die Gesamtkonzentration bevorzugt bei 0,5-5%, besonders bevorzugt bei 1-2% liegt.

Das erfindungsgemäße Flüssigpflaster kann darüber hinaus bevorzugt mit einem Farbstoff und/oder Duftstoff versetzt sein. Hierfür geeignete, entsprechend nicht-toxische, hautverträgliche Stoffe sind dem Fachmann bekannt. Die Konzentration dieser sollte hierbei etwa 10% nicht übersteigen.

Die Aushärtung erfolgt entweder rein passiv, d.h. ohne weiteres Zutun nach der Auftragung des flüssigen Pflasterfilms, oder aktiv, indem entweder durch Temperatureinwirkung und/oder Lichteinstrahlung die Filmbildung angeregt, unterstützt und/oder beschleunigt wird. Hat das Flüssigpflaster nur eine Komponente aus in Lösungsmittel aufgelöstem Filmbildner, so kann die Aushärtung passiv durch Verdunstung des Lösungsmittels erfolgen. Dies kann durch zusätzliche aktive Erwärmung beschleunigt werden. Zusätzlich kann durch Einstrahlung von Licht die Bildung von Kovalenten Bindungen zwischen Polymeren angeregt werden. Die ist insbesondere bei Polymeren mit C=C Doppel- oder Dreifachbindungen besonders einfach der Fall, da diese durch Photonen mit ca. 2,7 bzw. 1,8 eV aufgebrochen werden können, was Wellenlängen von 460 bzw. 688nm entspricht und damit im sichtbaren Bereich liegt. Aber auch Einfachbindungen können aufgebrochen werden, wenn UV Licht zum aktiven Aushärten verwendet wird.

Die Möglichkeit, die Vernetzung aktiv durch Lichteinstrahlung anzustoßen und zu kontrollieren ermöglicht auch lösungsmittelfreie einkomponentige Flüssigpflaster. Hierbei werden kurzkettige und/oder weniger verzweigte Polymere und/oder Polymere mit weniger polaren, insbesondere Hydroxyl, Gruppen eingesetzt, die im relevanten Temperaturbereich bei Standard-Atmosphärendruck flüssig sind und die gewünschte Viskosität von etwa 5-10Millipascalsekunden aufweisen. Mittel der erfindungsgemäßen Auftragevorrichtung werden diese auf die Wunde gebracht und verteilt. Dabei kann sich der Anwender so viel Zeit lassen, wie gewünscht, da die Vernetzung und damit Aushärtung erst durch Lichteinstrahlung in Gang gesetzt wird. Bei üblicher Umgebungsstrahlungsdichte läuft dieser Vorgang nur langsam ab, insbesondere wenn die Vernetzung auf dem Aufbrechen hochenergetischer Einfachbindungen beruht. Ist eine gewünschte Form und/oder Struktur erreicht, wird die Aushärtung durch Einstrahlung künstlichen Lichtes durchgeführt.
Da Hilfsmittel in Form einer Lichtquelle benötigt werden ist diese Art erfindungsgemäßer Flüssigpflaster insbesondere für den stationären Einsatz interessant.

Eine weitere mögliche Ausgestaltung des erfindungsgemäßen Flüssigpflasters sieht vor, zwei der Komponenten einzusetzen, die nur gemeinsam nach Mischung eine filmbildende Eigenschaft aufweisen. In Analogie zu Reaktionsharzen enthielte eine Komponente hierbei den eigentlichen Filmbildner, während die andere einen Härter umfasst, welcher nach Mischung mit dem Filmbildner die Polymerisation und Vernetzung anregt. Indem die Konzentration des Härters niedrig gehalten wird, kann die Härte des gehärteten Films wie gewünscht eingestellt werden.
Die erfindungsgemäße Auftragevorrichtung ist in ihren mehrkammerigen Ausführungsformen explizit auf den Einsatz solcher Mehrkomponenten-Flüssigpflaster ausgelegt. Die Mischung der beiden Komponenten erfolgt dabei vorteilhaft erst in der Spitze, welche leicht von ausgehärteten Rückständen zu reinigen und/oder als günstiges Verbrauchsteil ausgelegt und leicht wechselbar ist.

Weitere Einzelheiten, Merkmale und Vorteile vorliegender Erfindung ergeben sich aus den im Folgenden anhand der Figuren näher erläuterten Ausführungsbeispielen. Diese sollen vorliegende Erfindung nur illustrieren und ihren Gegenstand in keiner Weise einschränken.

Es zeigen im Einzelnen:
Figur 1: Querschnitt durch eine Ausführungsform einer erfindungsgemäßen Auftragevorrichtung
Figur 2: Querschnitt durch eine mehrkammerige Ausführungsform einer erfindungsgemäßen Auftragevorrichtung
Figur 3: In zwei Teilfiguren einen Längsschnitt und Querschnitte durch eine vierkammerige Auftragevorrichtung gemäß vorliegender Erfindung
Figur 4: In drei Teilfiguren mögliche Ausgestaltungen der Auftragespitze der erfindungsgemäßen Auftragevorrichtung.

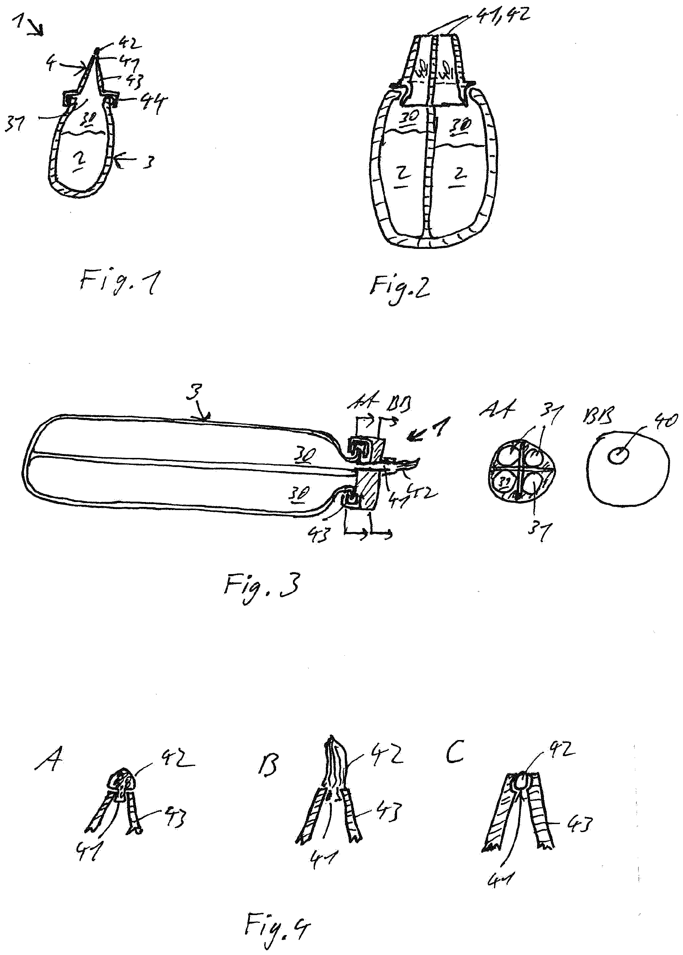

Figur 1 zeigt einen Querschnitt durch eine bevorzugte Ausführungsform der erfindungsgemäßen Auftragevorrichtung mit Flüssigpflaster. Sie besteht wie dargestellt aus dem Reservoir 3 in dessen Kammer 30 ein einkomponentiges Flüssigpflaster 2 eingefüllt ist. Am oberen Ende des Reservoirs ist Auftragemittel 4 aufgeschraubt. Dieses besteht aus einem Gewindekragen 44 an den sich nach oben ein hohler, kegelstumpfförmiger Korpus 43 anschließt. Am oberen Ende besitzt Korpus 43 eine Auftrageöffnung 41, in welche die Auftragespitze 42 leicht lösbar eingesteckt ist. Spitze 42 und Korpus 43 werden von einer Kappe 45 überdeckt, welche die Verdunstung des Flüssigpflasters und ggf. insbesondere des Lösungsmittels verhindert bzw. sehr stark verzögert.

Figur 2 zeigt einen Querschnitt durch eine mehrkammerige Ausführungsform einer erfindungsgemäßen Auftragevorrichtung. Im Inneren des Reservoirs 3 sind Trennwände 32 vorhanden, die das Innere in mehrere Kammern 30 unterteilen. Jede Kammer beherbergt eine Komponente eines Flüssigpflasters und hat eine eigene Öffnung 31 zum auf das Reservoir 3 aufgesteckten Auftragemittel 4, dessen Korpus 43 im Inneren den Kammern 30 zugeordnete Unterteilungen aufweist. Dadurch wird erreicht, dass die Komponenten des Flüssigpflasters 2 das Auftragemittel 4 getrennt durchfliesen und sich erst nach dem Ausfließen aus der hier die Auftragespitze 42 bildenden Auftrageöffnung 41 vereinigen und mischen.

Figur 3 zeigt einen Querschnitt durch eine vierkammerige Ausführungsform einer erfindungsgemäßen Auftragevorrichtung mit Flüssigpflaster. Das längliche, stiftartige Reservoir 3 hat im Inneren vier Kammern 30 mit einkomponentigem Flüssigpflastern unterschiedlicher Sekundäreigenschaften, wie Farbe oder Geruch. Jede Kammer verfügt wieder über eine eigene Öffnung 31 am oberen Ende. Das auf das Reservoir 3 aufgesteckte Auftragemittel 4 besteht aus einem relativ zum Steckkragen 44 drehbaren Korpus 43, der hier als Stufenzylinder gezeigt ist. Andere Formen sind jedoch ebenfalls denkbar. Das wesentliche ist, dass Kanal 40 durch Auftragemittel 4 bei korrekter Einstellung nur immer mit einer Öffnung des Reservoirs in Kommunikation steht. Rückmeldung über die korrekte Einstellung des Korpus wird dem Anwender durch Einrasten der Dichtlippe 46 am unteren Ende von Kanal 40 in einer der Kammeröffnungen 31 gegeben. Dazu ist Dichtlippe 46 aus ausreichend flexiblem Material insbesondere Gummi oder Kunststoff. In die Auftrageöffnung 41 eingesteckt und leicht wechselbar ist die Auftragespitze 42, hier in Form eines Pinsels dargestellt. Andere Ausführungsformen der Auftragespitze, etwa die in Figur 4 dargestellten, sind jedoch ebenso denkbar.

Figur 4 zeigt in drei Teilfiguren mögliche Ausgestaltungen der Auftragespitze der erfindungsgemäßen Auftragevorrichtung für ein kegelstumpfförmiges Auftragemittel ohne interne Unterteilungen. In Teilfigur A ist die Auftragespitze 42 durch eine schwammartige Struktur gebildet. Wird die erfindungsgemäße Auftragevorrichtung auf den Kopf gestellt fließt entweder rein Schwerkraftgetrieben oder durch zusätzlichen leichten Druck auf das Reservoir, falls dieses aus flexiblem Material besteht, Flüssigpflaster aus dem Reservoir durch dessen Öffnung 31 und den Korpus 43 des Auftragemittels 4 in die schwammartige Auftragespitze 42. Dort ist das Flüssigpflaster ohne weitere Krafteinwirkung durch die Kapillarkräfte infolge seiner endlichen Oberflächenspannung in den Poren gehalten. Diese bilden somit selbst eine Art temporäres Reservoir, welches durch Aufdrücken der Spitze 42 auf eine zu bedeckende Hautstelle geleert wird. Dadurch benetzt das Flüssigpflaster auf der Hautstelle und verteilt sich dort in gewissem Umfang. Dieser Vorgang kann durch Bewegung mit der Spitze unterstützt und gesteuert werden. Damit ähnelt die Verwendung dieser Ausführungsform der erfindungsgemäßen Auftragevorrichtung der eines Filzstiftes oder Markers. Die Oberflächenspannung des erfindungsgemäßen Flüssigpflasters ist so eingestellt, dass gute Benetzungseigenschaften sichergestellt sind. Die Viskosität ist nicht zu gering, sodass das Flüssigpflaster nicht ungewollt weiter verläuft, was, ein präzises Auftragen ermöglicht. Die Hydrophilie ist dabei ausreichend, um auch ein benetzen von feuchten Hautstellen, etwa nässenden Wunden zu gewährleisten.
Nach einer Verwendung verdunstet das Lösungsmittel nur langsam aus den mit Flüssigflasters gefüllten Poren der schwammartigen Spitze. Dies kann durch Kombination mit einer dicht umschließenden Kappe 45 noch weiter verlängert werden, sodass das Pflaster in der Spitze für lange Zeit flüssig bleibt. Jedoch selbst im Falle eines vollkommenen Eintrocknens der Spitze kann diese weiter verwendet werden. Denn mit dem trocknen ist eine Volumenreduktion verbunden, weswegen die Poren zumindest teilweise für Flüssigpflaster aus dem Reservoir zugänglich bleiben. Die darin enthaltenen Lösungsmittel verflüssigen den verfestigten Film und helfen beim Reinigen. Zusätzlich kann einfach durch mehrmaliges festes Aufdrücken auf eine stabile Oberfläche der Film aufgesprengt und entfernt werden. Alternativ ist auch ein Auswaschen mit einem der im Flüssigflasters verwendeten, durchweg hautverträglichen, Lösungsmittel möglich.

In Teilfigur B ist an Stelle eines Schwamms ein Pinsel als Auftragespitze 42 verwendet. Aufgrund der fein auslaufenden Spitze ist ein noch genaueres Verteilen des Flüssigpflasters nach dem Aufbringen möglich als bei der filzschreiberartigen Ausführungsform nach Teilfigur A.

In Teilfigur C besteht die Auftragespitze aus einer Kugel 42, welche in einer die Kugel teilweise Umschließenden Depression der Innenseiten des oberen Endes des kegelstumpfförmigen Korpus 43 gehalten ist. Beim Auftragen wird zunächst die Unterseite der Kugel 42 benetzt. Rollt der Anwender die Kugel der Auftragespitze 42 sodann über die Haut fließt das Flüssigpflaster auf die Haut aus und wird dort verteilt. Der große Vorteil dieser Ausführungsform ist die einfache Reinigung. Eingetrockneter Flüssigpflasterfilm kann per Hand und/oder kurzes Hin-und-Her-Rollen mit fest aufgedrückter Spitze 42 leicht entfernt werden.

### Bezugszeichenliste

- 1: Auftragevorrichtung
- 2: Flüssigpflaster
- 3: Reservoir
- 30: Kammer
- 31: Öffnung
- 4: Auftragemittel
- 40: Kanal
- 41: Auftrageöffnung
- 42: Auftragespitze
- 43: Korpus
- 44: Kragen
- 45: Kappe
- 46: Dichtlippe

## Patentansprüche

1. Auftragevorrichtung für Flüssigpflaster, umfassend:
- Flüssigpflaster (2) aus einer oder mehreren Komponenten, wobei das Flüssigpflaster nach Auftragen auf eine Hautstelle eines Anwenders durch passive oder aktive Aushärtung einen geschlossenen Film bildet und in seinen rheologischen Eigenschaften auf die Auftragevorrichtung abgestimmt ist,
- ein Reservoir (3) für das Flüssigpflaster (2) aus festem, durch keine der Inhaltsstoffe des Flüssigpflasters angreifbarem Material, wobei das Reservoir als ein Hohlkörper mit einer Öffnung (31) ausgebildet ist,
**dadurch gekennzeichnet,**
- **dass** auf der Öffnung (31) des Reservoirs (3) ein Auftragemittel (4) mit einer Auftragespitze (42) befestigt ist.

2. Auftragevorrichtung nach Anspruch 1, **dadurch gekennzeichnet, dass** das Auftragemittel (4) über ein unteres und ein oberes Ende verfügt, wobei es mit seinem unteren Ende auf der Öffnung (31) des Reservoirs (3), insbesondere durch Schrauben oder Stecken, dicht schließend befestigt ist und einen Korpus (43) mit einer Auftrageöffnung (41) umfasst, in und/oder jenseits derer sich die Auftragespitze (42) befindet.

3. Auftragevorrichtung nach Anspruch 2, **dadurch gekennzeichnet, dass** das Auftragemittel (4) ferner
- mehr als eine Auftrageöffnung (41), insbesondere für jede Komponente (21) des Flüssigflasters eine separate Auftrageöffnung (41), und/oder
- eine verschließende Kappe (45), und/oder
- einen Mechanismus, mittels dessen eine Auftrageöffnung (41) mit einer gewünschten Kammer (30) des Reservoirs (3) in fluide Kommunikation bringbar ist
umfasst.

4. Auftragevorrichtung nach Anspruch 3 mit mehr als einer Auftrageöffnung, **dadurch gekennzeichnet, dass** der Korpus (43) in seinem Inneren eine Unterteilung umfasst, von denen jede mit einer Kammer (30) des Reservoirs (3) sowie einer Auftrageöffnung (41) der Auftragespitze (42) in fluider Kommunikation steht.

5. Auftragevorrichtung nach einem der vorhergehenden Ansprüche, **dadurch gekennzeichnet, dass** das Auftragemittel (4) als an zwei gegenüberliegenden Enden offener Hohlkörper, insbesondere als hohler Kegelstumpf oder Hohlzylinder, ausgebildet ist, wobei es mit einem unteren Ende auf der Öffnung (31) des Reservoirs befestigt ist und an seinem oberen Ende ein oder mehrere Auftrageöffnungen (41) mit darüber angebrachter Auftragespitze (42) ausgebildet ist.

6. Auftragevorrichtung nach einem der vorhergehenden Ansprüche, **dadurch gekennzeichnet, dass** die Auftragespitze (42) eine Kugel, einen Pinsel oder einen Schwamm umfasst, wobei ein Material einer Oberfläche der Auftragespitze eine niedrige Adhäsion an das Flüssigpflaster im flüssigen und/oder ausgehärteten Zustand aufweist und/oder die Auftragespitze (42) leicht wechselbar ist.

7. Auftragevorrichtung nach einem der vorhergehenden Ansprüche, **dadurch gekennzeichnet, dass** das Reservoir (3)
- für jede der Komponenten des Flüssigpflasters (2) eine eigene Kammer (30) aufweist, und/oder
- zumindest teilweise aus transparentem Material, insbesondere, Glas, Acryl, Polycarbonat oder PET besteht, und/oder
- aus einem flexiblen und/oder bruchfesten Material, insbesondere einem Kunststoff, besteht.

8. Flüssigpflaster zur Verwendung in einer Auftragevorrichtung gemäß einem der Ansprüche 1 bis 7,
**dadurch gekennzeichnet, dass**
es eine Komponente mit einer filmbildenden Substanz, insbesondere Polysiloxan oder Polyethylen, umfasst und
- es aus genau einer Komponente besteht, und/oder
- aus mehr als einer Komponente besteht, wobei Aushärtung, insbesondere auf der Haut, nur nach Mischung aller Komponenten möglich ist, und/oder
- eine Komponente mit Mikro- oder Nanopartikeln umfasst, wobei die Partikel eine luftdurchlässige Struktur aufweisen und sich bei steigender Konzentration zusammenlagern, und/oder
- eine Komponente umfasst, die eine Suspension aus mehreren, nicht-mischbaren Lösungsmitteln unterschiedlicher Polarität u. Siedetemperaturen bei Normaldruck, insbesondere Heptan und Methanol oder Wasser und Ethylacetat, enthält, und/oder
- eine Komponente mit einem Lösungsmittel mit einem Polaritätsindex von weniger als 3 oder einem Dipolmoment von weniger als 1,3 Debye umfasst, und/oder
- eine Komponente mit einer antiseptischen und/oder einer wundheilungsfördernden Substanz umfasst, und/oder
- eine Komponente mit einem Farbstoff und/oder einem Duftstoff umfasst.

9. Flüssigpflaster nach Anspruch 8, **dadurch gekennzeichnet, dass** es nach Mischung aller Komponenten im Temperaturbereich zwischen 20 und 40 Grad Celsius
- eine Viskosität im Bereich von 0,2 bis 20, bevorzugt 1 bis 5 Pascalsekunden, und
- eine Oberflächenspannung zwischen ca. 20 und 40, bevorzugt 20 und 30, Millinewton pro Meter
aufweist.

10. Flüssigpflaster nach einem der Ansprüche 8 oder 9, **dadurch gekennzeichnet, dass** die Aushärtung
- passiv, insbesondere durch Verdunstung eines Lösungsmittels oder thermisch aktivierte Reaktion zwischen Substanzen aus einer Komponente oder aus verschiedenen Komponenten des Flüssigpflasters nach deren Mischung beim Auftragen, und/oder
- aktiv, insbesondere durch Erwärmen über eine Schwelltemperatur welche höher ist als die normale Hauttemperatur, jedoch bevorzugt 50Grad Celsius nicht übersteigt oder durch Bestrahlung mit Licht einer bestimmten spektralen Zusammensetzung
erfolgt.

11. Verwendung der Auftragevorrichung gemäß einem der Ansprüche 1-7 mit dem Flüssigpflaster gemäß einem der Ansprüche 8-10 zum Überdecken einer offenen Hautstelle, insbesondere einer Verletzung, eines Anwenders die mithilfe weiterer Mittel gereinigt und desinfiziert wird, mit einem fest haftenden, flexiblen, schmutz- und wasserdichten Film,
**gekennzeichnet durch**
die Schritte
i. ggf. Schütteln der Auftragevorrichtung (1) zur homogenen Durchmischung aller Komponenten (21) des Flüssigpflasters (2),
ii. ggf. Entfernen einer Kappe (45),
iii. Aufsetzen oder unmittelbares Darüberhalten der Auftragespitze (42) auf/über die Hautstelle,
iv. Ausbringen des Flüssigpflasters durch Schwerkraft oder zusätzlichen leichten Druck auf das Reservoir (3),
v. Verteilen des Flüssigpflasters durch natürliches Fließen ggf. aktiv unterstützt mittels der Auftragespitze (42), so dass ein in etwa gleichmäßig dicker Film einer gewünschten entsteht,
vi. aktives oder passives Aushärten/Aushärten-Lassen des Flüssigpflasterfilms.

12. Verwendung der Auftragevorrichtung gemäß einem der Ansprüche 1-7 mit dem Flüssigpflaster gemäß einem der Ansprüche 8-10 zum schmutzdichten Überdecken einer bereits mit gehärtetem Flüssigpflasterfilm bedeckten Hautstelle, insbesondere einer Verletzung, eines Anwenders,
**dadurch gekennzeichnet, dass**
a. der gehärtete Film schonend entfernt wird, und
b. dann die Schritte iii- vii gemäß Anspruch 11 durchgeführt werden.
